# EUROPEAN PATENT APPLICATION

(11) **EP 4 752 210 A1**
(43) Date of publication of application: **03.06.2026**
(21) Application number: 24848958.5
(22) Date of filing: 19.07.2024
(51) Int. Cl.: C12M 1/26

(54) **CELL TRANSFER DEVICE**

(30) Priority: 31.07.2023 JP 2023124538
(71) Applicant: Yamaha Hatsudoki Kabushiki Kaisha, Iwata-shi, Shizuoka 438-8501 (JP)
(72) Inventor: ITO, Saburo, Iwata-shi, Shizuoka 438-8501 (JP)
(74) Representative: Müller-Boré & Partner Patentanwälte PartG mbB
(86) International application number: PCT/JP2024/025937
(87) International publication number: WO 2025/028303

(57) **Abstract**

This cell transfer device is provided with: a camera that captures an image of a cell accommodated in a first container; a head that performs an operation of moving to the position of a target cell in the first container based on the image captured by the camera, causing the chip to suction the target cell, moving to a second container, and then causing the target cell to be discharged to the second container; and an information processing unit that corrects the position coordinates at which the head is actually present. The information processing unit: operates the head so that, with a predetermined location in a calibration area in the first container being a target coordinate position to which the head is to move, the chip gives a predetermined trace to the calibration area; causes the camera to capture an image of an area including the trace to identify an actual coordinate position of the trace; and determines a difference between the target coordinate position and the actual coordinate position to obtain a correction value for the position coordinates of the head.

## Description

### Technical Field

The present invention relates to a cell transfer device that transfers a specific target cell among cells accommodated in a first container to a second container.

### Background Art

For example, in the fields of medical and biological research, an operation of transferring cells from a culture container for culturing cells such as single cells and cell colonies to a work container for performing inspection, observation, and the like may be performed. For this operation, there is known a cell transfer device that performs an operation of picking and holding target cells from the culture container by a head to which a suction chip is attached, and releasing the held cells to the working container.

In picking the cells, it is necessary to accurately position the target cells and the tip opening of the suction chip. For example, when the tip opening accesses a position different from the XY coordinate position of the target cell or does not reach the cell culture surface, the target cell cannot be picked. In addition, if the tip opening is excessively pressed against the cell culture surface, the chip is damaged. Patent Literature 1 discloses a method of positioning a target cell and a tip opening of a suction chip by performing control to make a gap between the suction chip and a projection image thereof zero.

However, in the method of Patent Literature 1, an illumination system that generates a projection image of the suction chip is additionally required, and the device becomes complicated and expensive. In addition, positioning of the tip opening with respect to the target cell cannot be accurately performed in units of microns only by positioning the suction chip and the projection image thereof.

### Citation List

### Patent Literature

Patent Literature 1: Japanese Patent No. 6173331

### Summary of Invention

An object of the present invention is to provide a cell transfer device capable of accurately picking a target cell.

A cell transfer device according to one aspect of the present invention is a cell transfer device that transfers a specific target cell among cells accommodated in a first container to a second container, the cell transfer device including a camera configured to capture an image of a cell accommodated in the first container, a head to which a chip having a tip that sucks and discharges cells is attached, the head performing an operation of transferring the target cell to a position of the target cell in the first container based on an image captured by the camera, sucking the target cell into the chip, then moving to the second container, and discharging the target cell in the chip into the second container, and an information processing unit that corrects position coordinates at which the head exists. The information processing unit is configured to execute setting a calibration area in the first container and operating the head so that the chip imparts a predetermined trace to the calibration area with a predetermined location of the calibration area as a target coordinate position to be a transfer destination of the head, and specifying an actual coordinate position of the trace by causing the camera to image an area including the trace, and obtaining a difference between the target coordinate position and the actual coordinate position to acquire a correction value of the position coordinate of the head.

### Brief Description of Drawings

[FIG. 1] FIG. 1 is a diagram schematically illustrating a configuration example of a cell transfer device according to an embodiment of the present invention.
[FIG. 2] FIG. 2A is a cross-sectional view of a chip attached to a head and a diagram illustrating a mechanism for movement of the chip and a mechanism for suction of the chip, FIG. 2B is a cross-sectional view of the chip during a suction operation, and FIG. 2C is an enlarged view of a main part of FIG. 2B.
[FIG. 3] FIG. 3 is a block diagram illustrating an electrical configuration of a cell transfer device.
[FIG. 4] FIGS. 4A and 4B are diagrams for explaining a problem of conventional cell picking.
[FIG. 5] FIG. 5 is a diagram illustrating setting processing of a calibration area in a first embodiment.
[FIG. 6] FIGS. 6A and 6B are diagrams for explaining a trace forming operation by pressing the tip of the chip against a cell distribution area.
[FIG. 7] FIGS. 7A and 7B are schematic diagrams illustrating another example of trace formation in a cell distribution area.
[FIG. 8] FIG. 8 is a diagram illustrating a specific example of horizontal movement of the chip in the trace forming operation to the cell distribution area.
[FIG. 9] FIG. 9 is a diagram illustrating another example of setting of a calibration area.
[FIG. 10] FIG. 10 is a flowchart illustrating cell picking processing.
[FIG. 11] FIG. 11 is a flowchart illustrating correction processing.
[FIG. 12] FIG. 12 is a flowchart illustrating correction processing.
[FIG. 13] FIG. 13 is a flowchart illustrating correction processing according to a modification.
[FIG. 14] FIGS. 14A to 14D are diagrams for explaining correction processing according to a second embodiment of the present invention.
[FIG. 15] FIGS. 15A and 15B are diagrams illustrating a preferable example of a tip portion of the chip.

### Description of Embodiments

Hereinafter, embodiments of the present invention will be described in detail with reference to the drawings. The cell transfer device according to the present invention can pick and transfer various biologically derived cells. Examples of the biologically derived cells include blood cells, cells such as singulated cells and fertilized eggs, small tissue pieces such as Histoculture, cell aggregates such as spheroids and organoids, individuals such as zebrafish and nematodes, and 2D or 3D cell colonies. In the present specification, the term "cell" includes these various cells. In particular, the cell transfer device of the present invention is suitable for picking and transferring cells such as single cells, cell aggregates, and cell colonies, which generally require picking under microscopic observation.

### [Overall Configuration of Cell Transfer Device]

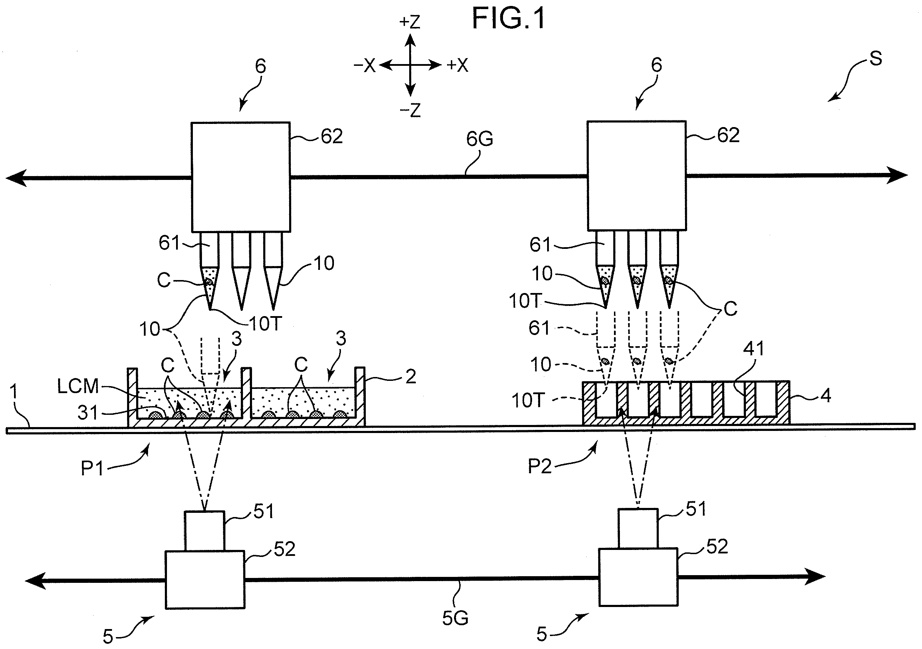

FIG. 1 is a diagram schematically illustrating an overall configuration of a cell transfer device S according to an embodiment of the present invention. Here, the cell transfer device S for transferring the cells C between two containers, that is, between a culture plate 2 (first container) and a transfer destination plate 4 (second container) is illustrated. Of course, the cell transfer device S may be configured to transfer the cells C between three or more containers.

The cell transfer device S includes a translucent base 1 having a horizontal placement surface, a camera unit 5 disposed below the base 1, and a head unit 6 disposed above the base 1. The culture plate 2, which is the transfer source of the cell C, is placed on a first placement position P1 of the base 1, and the transfer destination plate 4, which is the transfer destination of the cell C, is placed on a second placement position P2. The head unit 6 includes a plurality of heads 61 that can be moved up and down in the Z direction (vertical direction). A chip 10 for sucking and discharging the cell C is attached to the lower end of each head 61. The camera unit 5 and the head unit 6 are movable in an X direction (horizontal direction) and a direction perpendicular to the paper surface of FIG. 1 (Y direction). The culture plate 2 and the transfer destination plate 4 are placed on the upper surface of the base 1 within a movable range of the head unit 6.

Generally, the cell transfer device S performs picking for individually sucking a specific target cell C by each of the plurality of chips 10 from the culture plate 2 in which a large number of cells C are cultured. Then, the picked cells C are transferred to the transfer destination plate 4, and the cells C are discharged from the plurality of chips 10 to the transfer destination plate 4 (well 41). Before the picking of the cells C, the cells C held in the culture plate 2 are imaged by the camera unit 5, and a selection operation of selecting high-quality cells C to be transferred to the transfer destination plate 4 is performed.

Hereinafter, each unit of the cell transfer device S will be described. The base 1 is a flat plate that has predetermined rigidity and is partially or entirely made of a translucent material. A preferred base 1 is a glass plate. By forming the base 1 with a translucent material such as a glass plate, the camera unit 5 disposed below the base 1 can image, through the base 1, the culture plate 2 and the transfer destination plate 4 disposed on the upper surface of the base 1.

The culture plate 2 is a container serving as a transfer source of the cells C, and includes a plurality of wells 3 for culturing the cells C. Each well 3 is a small container having an upper surface opening, and has a flat bottom surface 31. The cultured cells C are located (adhered or floating) on the bottom surface 31. Into the well 3, liquid medium LCM is injected, and test cells are seeded. For the culture plate 2, a member made of a translucent resin material or glass is used in order to enable imaging of the cell C by the camera unit 5 disposed below. As the culture plate 2, for example, a commercially available 6-well plate (for example, model number 3516 manufactured by Corning Incorporated) can be used.

The transfer destination plate 4 has a plurality of wells 41 to which the cells C picked from the culture plate 2 are discharged. The well 41 is a bottomed hole opened in the upper surface of the transfer destination plate 4. In one well 41, a required number (usually 1) of cells C are accommodated together with a liquid culture medium. Various tests such as addition of a reagent or a reactant, observation, culture, and the like are performed on the cells C accommodated in the well 41. As the transfer destination plate 4, a member made of a translucent resin material or glass is used. As the transfer destination plate 4, for example, a commercially available 96-well plate (for example, model number 3595 manufactured by Corning Incorporated) can be used.

The camera unit 5 is a device that captures an image of the cell C held by the culture plate 2 or the transfer destination plate 4 from the lower surface side of the culture plate 2 or the transfer destination plate 4, and includes a lens portion 51 and a camera body 52. The lens portion 51 is an objective lens used in an optical microscope and includes a lens group that forms an optical image of a predetermined magnification and a lens barrel that accommodates the lens group. The camera body 52 includes an imaging element such as a CCD image sensor. The lens portion 51 forms an optical image of an imaging object on a light receiving surface of the imaging element. The camera unit 5 is movable in the X direction and the Y direction below the base 1 along a guide rail 5G extending in the left-right direction in parallel with the base 1. Furthermore, the lens portion 51 is movable in the Z direction for focusing operation.

The head unit 6 is a device provided for picking the cells C from the culture plate 2 and transferring the cells C to the transfer destination plate 4. The head unit 6 includes a plurality of heads 61 and a head body 62 to which the heads 61 are assembled. The chip 10 for sucking and discharging the cell C is attached to the tip of each head 61. The head body 62 holds the head 61 so as to be movable up and down in the +Z and -Z directions (vertical direction), and is movable in the +X and -X directions (horizontal direction) along a guide rail 6G. The head body 62 is also movable in the Y direction. That is, the head 61 is movable in a three-dimensional direction of XYZ.

The general operation of the head 61 is as follows. The head 61 to which the chip 10 is attached is moved in the XY directions to a coordinate position corresponding to the position of the target cell C in the well 3 (first container) to be moved, which is specified based on the image captured by the camera unit 5. Subsequently, the head 61 is lowered to approach the target cell C. After the target cells C are sucked into the chip 10, the head 61 is moved in the XY directions to the transfer destination plate 4 (second container) after being raised. The target cells C in the chip 10 are discharged to a predetermined well 41.

### [Details of Chip and Head]

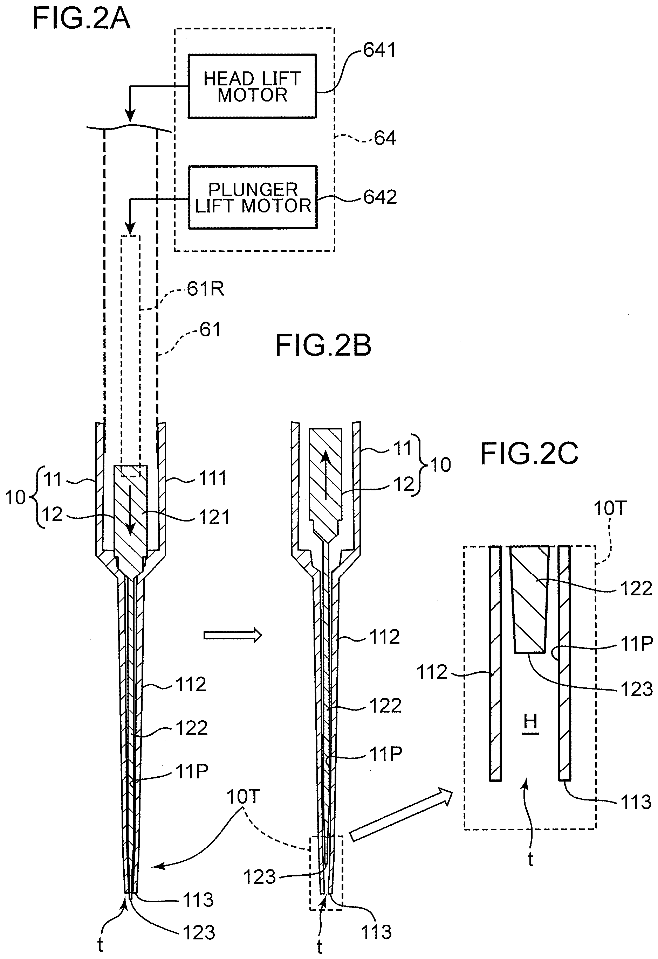

FIG. 2A is a cross-sectional view of the chip 10 attached to the head 61, and a diagram illustrating a mechanism for movement of the chip 10 and a mechanism for suction of the chip 10. The chip 10 is a tool for sucking or discharging the cells C for moving the cells C, and includes a tip portion 10T having a tip opening t through which the cell C can enter and exit. The chip 10 of the present embodiment includes an assembly of a syringe 11 and a plunger 12. The syringe 11 includes a tubular passage 11P serving as a suction path of the cell C therein. The plunger 12 advances and retreats in the tubular passage 11P while being in sliding contact with the inner peripheral wall of the syringe 11 defining the tubular passage 11P.

The syringe 11 includes a syringe base end 111 formed of a large-diameter cylindrical body and a syringe body 112 formed of a small-diameter long cylindrical body. The tubular passage 11P is formed in the syringe body 112. The above-described tip opening t is provided at the syringe tip portion 113 (tip of the chip) which is the lower end of the syringe body 112. One end of the tubular passage 11P is connected to the tip opening t. The syringe base end 111 is connected to the other end side of the syringe body 112 via a tapered portion. An upper end portion of the syringe base end 111 is fitted and attached to a lower end of the head 61.

The plunger 12 includes a plunger base end 121 made of a cylindrical body, a needle-shaped plunger body 122 continuous below the plunger base end 121, and a plunger tip portion 123 which is a lower end of the plunger body 122. The plunger 12 is assembled to the syringe 11 in an aspect in which the plunger base end 121 is accommodated in the syringe base end 111 and the plunger body 122 is inserted into the tubular passage 11P of the syringe body 112. In the state of FIG. 2A in which the plunger body 122 is inserted through the syringe body 112 at the deepest position, the plunger tip portion 123 protrudes from the tip opening t. A rod 61R movable in the vertical direction in the internal space of the head 61 is attached to the upper end of the plunger base end 121.

The head body 62 is equipped with a head drive unit 64. The head drive unit 64 functions as a mechanism for moving the chip 10 attached to the head 61 in the vertical direction and a mechanism for sucking and discharging the cell C into and from the chip 10 through the tip opening t of the chip 10. The head drive unit 64 includes a head lift motor 641 and a plunger lift motor 642.

The head lift motor 641 is a motor serving as a drive source that moves the head 61 up and down with respect to the head body 62. When the head 61 is moved up and down by driving of the head lift motor 641, the chip 10 attached to the lower end of the head 61 is also moved up and down. That is, the height position of the tip opening t of the tip portion 10T can be set to a desired position by operation control of the head lift motor 641.

The plunger lift motor 642 is a motor serving as a drive source that moves the rod 61R up and down in the internal space of the head 61. When the rod 61R is moved up and down, the plunger 12 attached to the rod 61R is also moved up and down. When the plunger 12 rises with respect to the syringe 11, a suction force is generated in the tip opening t. On the other hand, when the plunger 12 is lowered with respect to the syringe 11, a discharge force is generated in the tip opening t. That is, the suction operation and the discharge operation of the cell C by the chip 10 can be controlled by the operation control of the plunger lift motor 642.

FIG. 2A illustrates a state in which the plunger 12 is lowered to the maximum. This state is a state before the cell C is sucked to the chip 10 or a state in which the sucked cell C is discharged from the chip 10. The plunger tip portion 123 protrudes slightly downward from the syringe tip portion 113. FIG. 2B illustrates a state in which the plunger 12 is raised by a predetermined height. This state is the state of the chip 10 at the time of the suction operation of sucking the cell C. FIG. 2C is an enlarged view of a main part of FIG. 2B.

During the suction operation, the plunger tip portion 123 retracts into the tubular passage 11P. At this time, a suction force is generated in the tip opening t, and the fluid around the tip opening t is sucked into a suction space H formed in the tubular passage 11P by the plunger tip portion 123 being retracted. That is, a medium LCM containing the cells C is held in the suction space H. After this suction operation, when the plunger 12 is moved downward, the fluid held in the suction space H is discharged from the tip opening t. The suction amount of the fluid can be adjusted by the rising height of the plunger 12, and the suction speed of the fluid can be adjusted by the rising speed of the plunger 12.

The chip 10 may be made of any of resin, metal, and glass. However, the chip 10 is desirably made of an elastically deformable member. As will be described in detail later, in the present embodiment, the tip portion 10T of the chip 10 may abut on the bottom surface 31 of the well 3 for tracing, and furthermore, the chip may be moved in the abutting state. When the chip 10 is elastically deformed, it is possible to reduce the possibility of breakage at the time of the abutment.

### [Electrical Configuration of Cell Transfer Device]

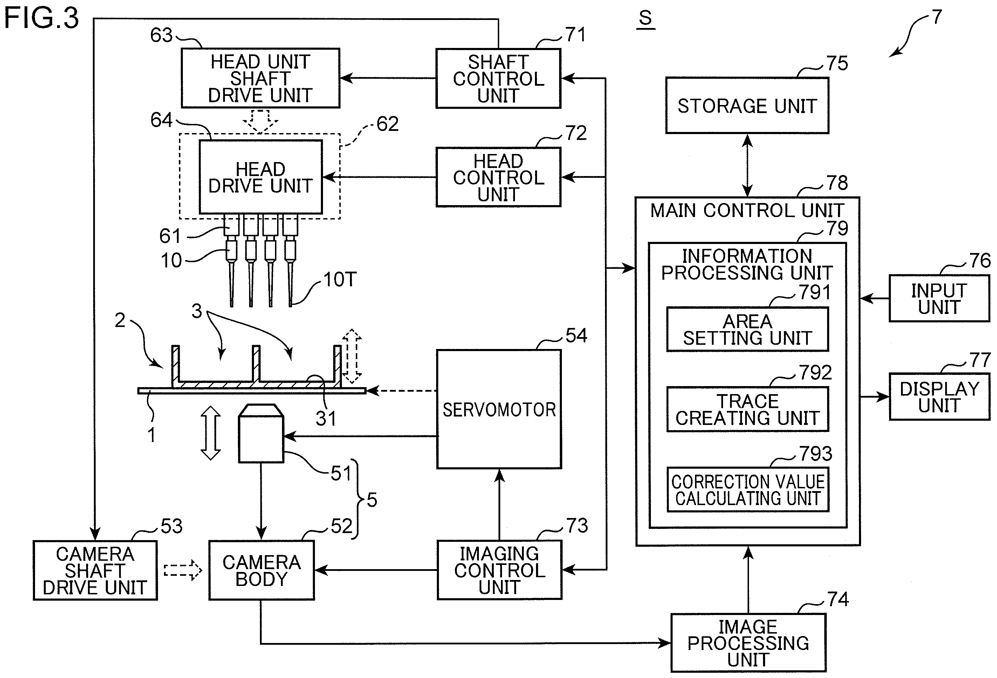

FIG. 3 is a block diagram illustrating an electrical configuration of the cell transfer device S. The cell transfer device S includes a control unit 7 that controls the movement of the head unit 6 (see FIG. 1) and the elevation and descent of the head 61 (chip 10), that is, the movement of the head 61 in the three-dimensional direction. In addition, the control unit 7 controls suction and discharge operations of the cells C to the chip 10, movement of the camera unit 5, imaging operations, and the like.

In addition, the cell transfer device S includes a camera shaft drive unit 53, a servomotor 54, a head unit shaft drive unit 63, and a head drive unit 64. The camera shaft drive unit 53 includes a drive motor that horizontally moves the camera unit 5 along the guide rail 5G (FIG. 1). The servomotor 54 rotates forward or backward to move the lens portion 51 in the vertical direction with a predetermined resolution via a power transmission mechanism (not illustrated). As a result, the focal position of the lens portion 51 is aligned with the cell C accommodated in the well 3. Note that, as indicated by a dotted line in FIG. 3, not the lens portion 51 but the base 1 side may be moved up and down. The head unit shaft drive unit 63 includes a drive motor that horizontally moves the head unit 6 (head body 62) in the X or Y direction along the guide rail 6G. The head drive unit 64 is as described above with reference to FIG. 2.

The control unit 7 includes a processor or the like, and functions to include a shaft control unit 71, a head control unit 72, an imaging control unit 73, an image processing unit 74, a storage unit 75, and a main control unit 78 by executing a predetermined program. Furthermore, an input unit 76 that inputs various types of information to the control unit 7 and a display unit 77 that displays various types of information are provided. The input unit 76 receives inputs of various types of operation information from the operator. The display unit 77 functions as a monitor that displays an image or the like captured by the camera unit 5.

The shaft control unit 71 controls the operation of the head unit shaft drive unit 63. The shaft control unit 71 moves the head unit 6 to a predetermined target position in the horizontal direction by controlling the head unit shaft drive unit 63. Movement of the head 61 (chip 10) between the culture plate 2 and the transfer destination plate 4, positioning vertically above the cells C accommodated in the wells 3, positioning vertically above the wells 41 to be discharged, and the like are realized by control of the head unit shaft drive unit 63 by the shaft control unit 71. In addition, the shaft control unit 71 controls the camera shaft drive unit 53 to control the operation of moving the camera unit 5 along the guide rail 5G. Furthermore, the operation of the head 61 for imparting a trace to the well 3 by the chip 10 described later is also controlled by the shaft control unit 71.

The head control unit 72 controls the head lift motor 641 of the head drive unit 64 to move the head 61 up and down to be controlled toward a predetermined target position. In addition, the head control unit 72 generates a suction force or a discharge force in the tip opening t of the chip 10 at a predetermined timing by controlling the plunger lift motor 642.

The imaging control unit 73 controls an imaging operation of the culture plate 2 or the transfer destination plate 4 by the camera unit 5, for example, an exposure amount, a shutter timing, and the like. Furthermore, the imaging control unit 73 gives a control pulse for moving the lens portion 51 in the vertical direction at a predetermined pitch (for example, a pitch of several tens µm) to the servomotor 54 for the focusing operation.

The image processing unit 74 performs image processing such as edge detection processing and pattern recognition processing accompanied by feature amount extraction on the image data acquired by the camera body 52. Based on the image of the culture plate 2 on which the cell C is cultured, the image processing unit 74 executes a process of recognizing the presence of the cell C on the bottom surface 31 and the trace imparted by the chip 10 on the image. Similarly, the image processing unit 74 executes a process of recognizing the number, amount, fluorescence intensity, and the like of the cells C accommodated in the well 41 based on the image of the well 41 to which the cells C have been moved.

The storage unit 75 stores various setting values, data, programs, and the like in the cell transfer device S. In addition, the storage unit 75 stores information regarding the culture plate 2 to be used, for example, data such as a plate size, a size of the well 3, and an uneven range of the bottom surface 31. In addition, information regarding the tip shape of the chip 10, for example, the outer diameter and thickness of the syringe tip portion 113, the opening diameter of the tip opening t, and the like are also stored in the storage unit 75. Further, setting information and the like such as the suction amount and the suction speed in the suction operation of the cell C and the moving pitch and the horizontal moving amount of the head 61 in the Z direction in the trace imparting control are also stored in the storage unit 75.

The main control unit 78 integrally controls the operations of the camera unit 5 and the head unit 6. The main control unit 78 controls the camera unit 5 and the head unit 6 through the shaft control unit 71, the head control unit 72, and the imaging control unit 73 so as to image the culture plate 2, perform picking to cause the chip 10 attached to the head 61 to suck the cell C selected as the movement target, and transfer the cell C to the transfer destination plate 4. Furthermore, in addition to such integral control, the main control unit 78 imparts a predetermined trace to the calibration area set on the bottom surface 31 of the well 3 or the like to the chip 10 through the head 61, and executes correction control for obtaining a correction value of the movement amount of the head 61 based on the trace. The calibration area is an area that can be accessed by the chip 10 in the well 3 (first container), and is set to an area in which interference with the target cell to be transferred does not occur at the time of the access.

The main control unit 78 functionally includes an information processing unit 79 for the above correction control. The information processing unit 79 executes a process of correcting the position coordinates at which the head 61 actually exists. The information processing unit 79 includes an area setting unit 791, a trace creating unit 792, and a correction value calculating unit 793. The area setting unit 791 performs a process of setting a calibration area to which a trace serving as a reference position is imparted in the correction control. The trace creating unit 792 operates the head 61 so that the chip 10 imparts a predetermined trace to the calibration area with a predetermined location of the calibration area as a target coordinate position to be a transfer destination of the head 61. The correction value calculating unit 793 performs a process of acquiring a correction value of the position coordinates of the head 61 by causing the camera unit 5 to image the region including the trace, specifying an actual coordinate position of the trace, and obtaining a difference between the target coordinate position and the actual coordinate position.

### [Problems in Cell Picking]

In order to accurately suck, that is, pick the target cell with the chip 10, it is important to accurately align the tip opening t of the chip 10 with the target cell. However, in actual cell picking, even if the head 61 is moved to target position coordinates, the picking of the target cells may fail. As factors that hinder the positioning, there are a positional shift of the tip portion 10T of the chip 10 in the state of being attached to the head 61 and a height variation of the bottom surface 31 of the well 3, which is the culture surface of the cell C.

The positional shift of the tip portion 10T is caused by a mounting position shift of the chip 10 with respect to the head 61, thermal expansion and contraction of the guide rail 6G which is a moving axis of the head 61, other mechanical errors, and a width of a focal depth in a case where a height position of the tip portion 10T is specified based on a camera image. As illustrated in FIG. 2A, the chip 10 is fitted into the lower end of the head 61, but an inevitable fitting error occurs. In addition, the influence of thermal expansion and contraction, a mechanical error, and a focal depth cannot be zero. That is, the position of the tip portion 10T of the chip 10 is shifted in the XYZ directions.

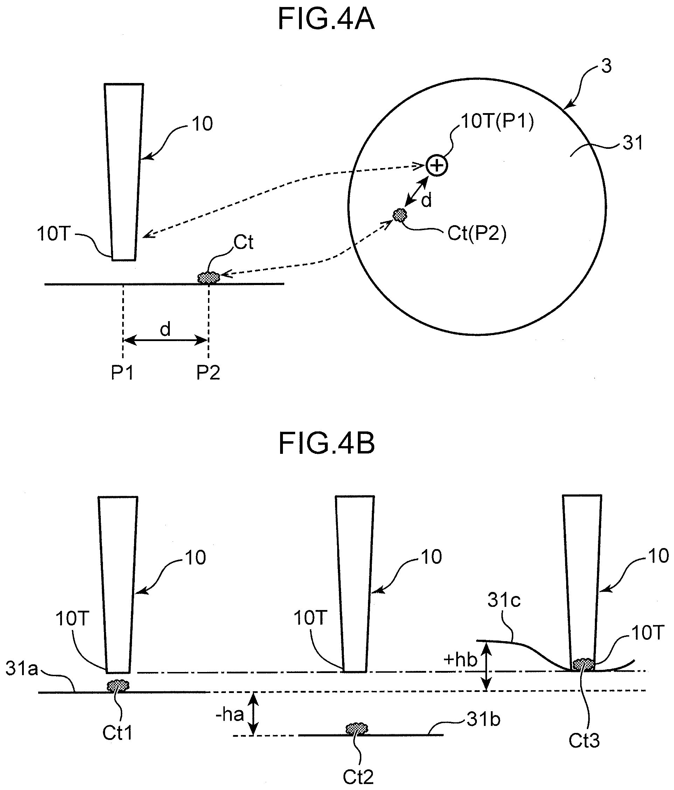

FIG. 4A is a diagram illustrating an occurrence situation of a cell picking mistake due to a positional shift of the tip portion 10T. It is assumed that a target cell Ct to be picked is in contact with the bottom surface 31 of the well 3. When the coordinate position of the target cell Ct is specified based on the photographed image of the camera unit 5, the head 61 (chip 10) is moved with the coordinate position as the target coordinate position. However, due to the positional shift in the XYZ directions, the XY position = P1 approached by the tip portion 10T becomes a position separated by a distance d with respect to the XY position = P2 where the target cell Ct exists. In addition, an error in the approach height may also occur in the Z direction. In such a situation, the picking of the target cell Ct by the chip 10 fails.

In addition, the bottom surface 31 of the well 3 has non-negligible variation in height, that is, surface irregularities. The thickness size of the cell C to be picked is, for example, about several µm to several tens µm. The bottom surface 31 is a macroscopically horizontal surface, but has surface irregularities in the order of several µm to several tens µm when observed in the order of microns. As a result of measurement by the present inventor, it has been confirmed that the height of the bottom surface 31 of the well 3 of the commercially available 6-well plate had a height difference of about 100 µm at the maximum among the 6 wells. When there is such a height variation of the bottom surface 31, even if the tip portion 10T of the chip 10 is lowered to a certain height position, the cell C may not be sucked.

Referring to FIG. 4B, it is assumed that the well 3 includes a first bottom surface 31a at a certain height position, a second bottom surface 31b at a position lower than the first bottom surface 31a by -ha, and a third bottom surface 31c at a position higher than the first bottom surface 31a by +hb. The first bottom surface 31a, the second bottom surface 31b, and the third bottom surface 31c are provided on the bottom surface 31 of one well 3, or are provided in one well 3 and another well 3 included in one culture plate 2.

In this situation, it is assumed that the lowering height of the tip portion 10T of the chip 10 is set to the height position of the first bottom surface 31a and the picking of the target cell Ct is executed. In this case, a cell Ct1 contacting with the first bottom surface 31a can be sucked from the tip opening t. However, a cell Ct2 contacting with the second bottom surface 31b cannot be sucked because the approach height of the tip portion 10T is insufficient by -ha. On the other hand, the tip portion 10T approaches the third bottom surface 31c excessively deeply by +hb. FIG. 4B illustrates a state in which the third bottom surface 31c is pressed against the tip portion 10T and deformed in a recessed manner, but the tip portion 10T is actually curved or damaged. For this reason, suction of a cell Ct3 in contact with the third bottom surface 31c often fails. The movement correction control of the head 61 according to the present embodiment aims to solve the above-described problem.

### [First Embodiment of Correction Control]

The correction control of the present embodiment roughly includes the following steps 1 to 3.
· Step 1; A calibration area is set in the well 3.
· Step 2; A predetermined trace is imparted to the calibration area with the chip 10.
· Step 3; The correction value of the coordinate position is acquired using the trace.

That is, the target coordinate position is imparted to the drive unit of the head 61 to operate the head 61, and the tip portion 10T of the chip 10 is caused to actually approach the calibration area in the well 3 to impart some trace. Then, the actual coordinate position actually approached by the tip portion 10T is specified from the trace, and a difference between the target coordinate position and the actual coordinate position is obtained. The correction value of the coordinate position to be the movement target of the head 61 is derived based on the deviation between the two. Hereinafter, steps 1 to 3 will be described in detail.

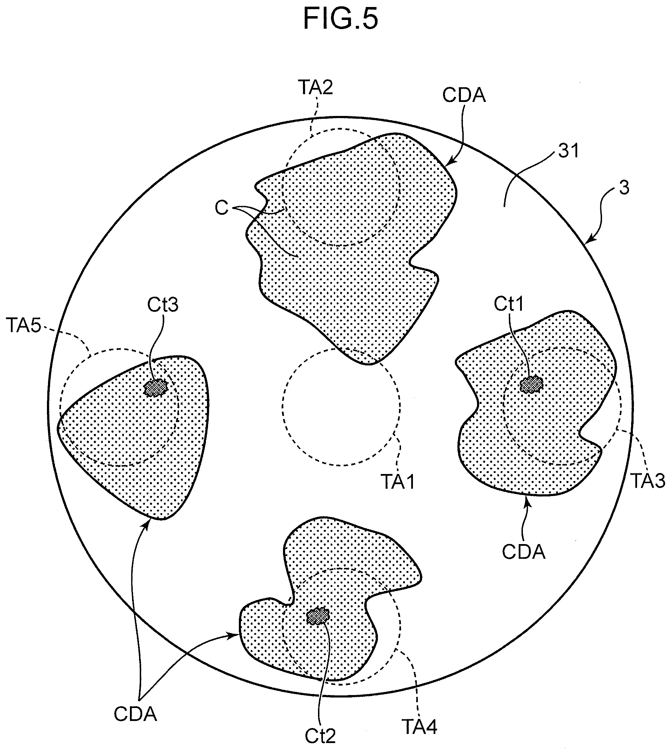

FIG. 5 is a diagram illustrating an example of the setting processing of the calibration area in step 1, executed by the area setting unit 791 of the information processing unit 79. It is assumed that a cell distribution area CDA in which cells are relatively densely gathered exists on the bottom surface 31 of the well 3 as a culture surface, and cells exist relatively sparsely in other areas. The area setting unit 791 sets, on the bottom surface 31, a calibration area to which the tip portion 10T of the chip 10 is actually approached in the correction control. FIG. 5 illustrates an example in which five circular chip correction positions TA1, TA2, TA3, TA4, and TA5 are set as the calibration area. The chip correction position TA1 is arranged at the center portion of the bottom surface 31, and the chip correction positions TA2 to TA5 are arranged at intervals of 90 degrees in the circumferential direction in the vicinity of the peripheral edge of the bottom surface 31. The chip correction positions TA1 to TA5 may be fixedly determined in advance, or may be determined according to the generation situation of the cell distribution area CDA on the bottom surface 31.

Among the five chip correction positions TA1 to TA5, the cell distribution areas CDA each including the target cells Ct1, Ct2, and Ct3 to be transferred partially overlap TA3, TA4, and TA5. For example, when the target cell Ct1 is picked, an area that is the cell distribution area CDA existing at the chip correction position TA3 and in which an unused cell group other than the target cell Ct1 exists is set as the calibration area. In the picking of the target cell Ct2, an area that is the cell distribution area CDA overlapping the chip correction position TA4 and in which a cell group other than the target cell Ct2 exists is set as the calibration area.

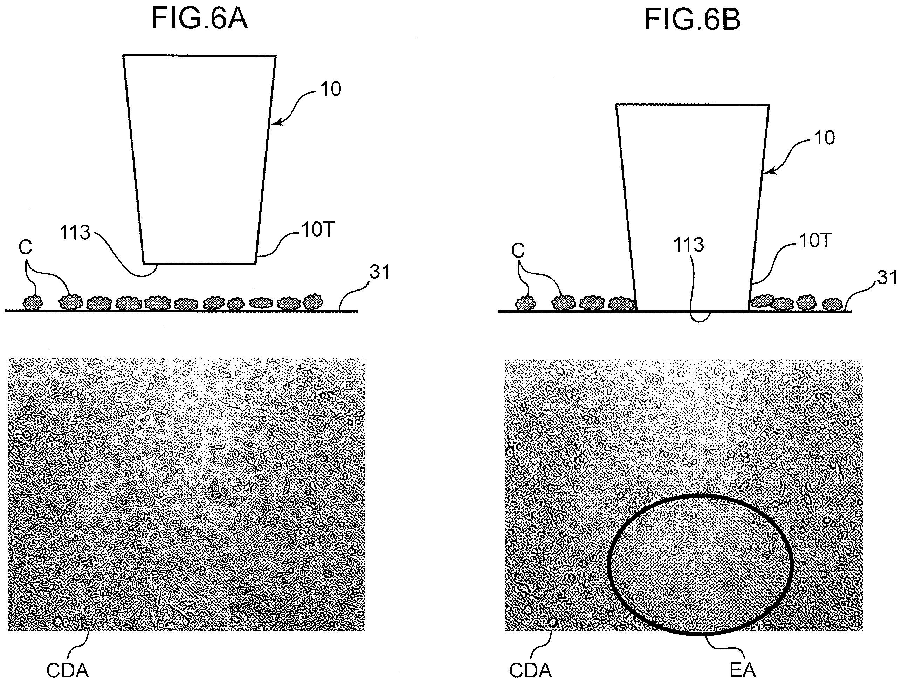

FIGS. 6A and 6B are diagrams illustrating an execution status of a trace imparting process in step 2 executed by the trace creating unit 792. The trace creating unit 792 presses the syringe tip portion 113, which is the tip of the chip 10, against the cell distribution area CDA, and controls the operation of the head 61 through the shaft control unit 71 so as to form a void portion EA as a trace in the cell distribution area CDA.

FIG. 6A illustrates a state in which the syringe tip portion 113 faces the cells C in the cell distribution area CDA on the bottom surface 31 in a non-contact manner. The upper view of FIG. 6B shows a state in which the syringe tip portion 113 is pressed against the bottom surface 31 in the cell distribution area CDA. The lower diagram of FIG. 6B illustrates a state in which the cell C is released to the periphery of the syringe tip portion 113 by the pressing, and as a result, a void portion EA in which the cell C hardly exists is formed in the cell distribution area CDA. The void portion EA formed in the cell distribution area CDA where a large number of cells C gather can be easily confirmed by a camera image. That is, a trace that is easily confirmed on the image can be formed by a simple operation of pressing the tip of the chip 10.

The trace creating unit 792 imparts a target coordinate position as a transfer destination of the head 61 to the shaft control unit 71 and moves the head in the XY directions. After the movement, the trace creating unit 792 lowers the head 61 at the first high speed until the syringe tip portion 113 is located at the predetermined opposing height. The first speed is a normal lowering speed or a maximum speed during operation of the head 61. The predetermined opposing height is set in consideration of an upper limit of a height dimensional tolerance of the bottom surface 31, a mechanical error of the head unit 6, and the like. The trace creating unit 792 lowers the head 61 at a second speed lower than the first speed after the predetermined opposing height. The lowering at the second speed includes an aspect of simply lowering the head 61 at a low speed, an aspect of stepwise lowering the head 61 at a predetermined pitch, and the like. In the latter aspect, the trace creating unit 792 gradually lowers the head 61 at a pitch of several µm, for example. The camera unit 5 is caused to image the cell distribution area CDA at every one pitch of the lowering, and the pressing trace of the chip 10 is confirmed. When the void portion EA illustrated in FIG. 6B is confirmed, the trace creating unit 792 raises the head 61. The first speed and the second speed may be the same speed. That is, the second speed may be equal to or less than the first speed. For example, in the above-described aspect in which the head 61 is lowered stepwise, the head 61 may be lowered stepwise while maintaining the lowering speed at the same speed as the first speed at the time of lowering after the predetermined opposing height.

The predetermined opposing height serving as a point at which the lowering aspect of the head 61 is switched from high-speed lowering to low-speed lowering (stepwise lowering) may be changed depending on whether the height position of the cell C can be accurately acquired. This is because a period during which the head 61 is stepwise lowered at a predetermined pitch and the imaging by the camera unit 5 is set is shortened as much as possible, and the process of imparting a trace is speeded up. There is a case where the height position of the cell C, that is, the Z coordinate can be grasped by imaging the cell distribution area CDA or the like up to the process of imparting a trace. In this case, the trace creating unit 792 sets the height position obtained by adding the upper limit of the mechanical error of the head unit 6 to the Z coordinate of the cell C to the predetermined opposing height. As a result, the chip 10 can be lowered at a high speed until immediately before the syringe tip portion 113 of the chip 10 presses against the cell C, which can contribute to improvement of tact.

On the other hand, although the Z coordinate of the cell C is estimated, the Z coordinate may not be accurately grasped. That is, the Z coordinate of the cell C has been temporarily acquired, but the accuracy is unknown. In this case, the trace creating unit 792 sets, to the predetermined opposing height, the height position obtained by adding the upper limit of the height dimensional tolerance of the bottom surface 31 and the upper limit of the mechanical error of the head unit 6 to the temporarily acquired Z coordinate of the cell C. For example, assuming that the height dimensional tolerance of the bottom surface 31 is ±100 µm and the mechanical error of the head unit 6 is ±50 µm, a height obtained by adding 150 µm to the Z coordinate of the temporarily acquired cell C is set as the predetermined opposing height. As a result, images before and after the syringe tip portion 113 is pressed against the cell C can be reliably acquired in the process of stepwise lowering of the head 61.

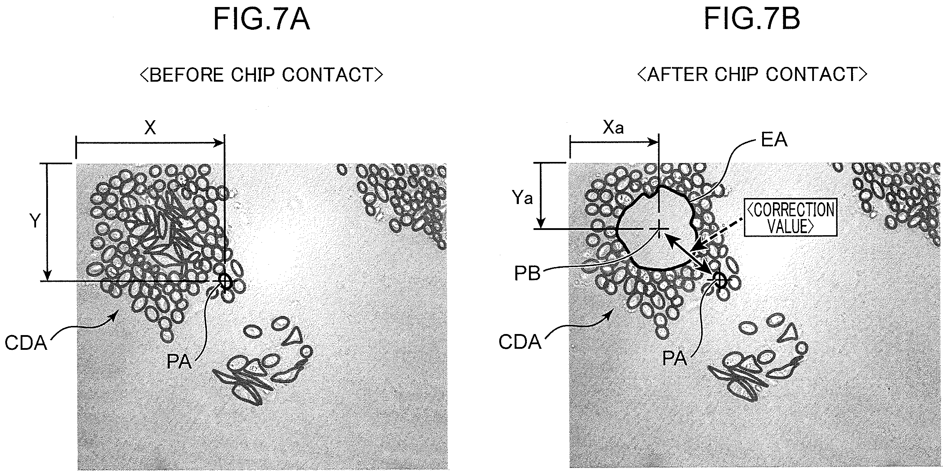

FIGS. 7A and 7B are diagrams schematically illustrating correction value acquisition processing in step 3 executed by the correction value calculating unit 793. The correction value calculating unit 793 obtains a movement correction value of the head 61 from a difference between the target coordinate position and the actual coordinate position of the void portion EA. In the cell distribution area CDA of FIG. 7A, a target coordinate position PA(X, Y) set by the trace creating unit 792 is illustrated. If there is no positional shift of the tip portion 10T of the chip 10, the syringe tip portion 113 faces the target coordinate position PA.

FIG. 7B illustrates the void portion EA actually formed in the cell distribution area CDA by imparting the target coordinate position PA to the shaft control unit 71 and operating the head 61 to press the tip of the chip 10. The void portion EA has a size corresponding to the outer diameter of the syringe tip portion 113. Therefore, the correction value calculating unit 793 obtains the center position of the void portion EA based on the image including the void portion EA, and handles the center position as an actual coordinate position PB. For example, processing can be applied in which the contour of the void portion EA is obtained by edge detection image processing, and the obtained contour is replaced with an approximate circle to obtain the center position.

In the example of FIG. 7B, the actual coordinate position PB(Xa, Ya) is shifted with respect to the target coordinate position PA(X, Y). The correction value calculating unit 793 obtains a difference between both coordinates, and sets the difference as a correction value of the XY movement of the head 61 on the XY plane. Note that the correction value of the head 61 in the Z direction orthogonal to the XY plane is acquired based on the Z-coordinate position where the chip 10 is pressed against the bottom surface 31, detected by the stepwise lowering of the chip 10 executed in step 2. Such a correction value is obtained for each well 3 and for each type of the chip 10.

The correction value calculating unit 793 may refer to the information regarding the tip shape of the chip 10 when specifying the void portion EA from the image including the void portion EA. As illustrated in FIG. 6B, if the cell distribution area CDA where many cells C are densely packed exists on the bottom surface 31 of the well 3, it is relatively easy to identify the void portion EA on the image. However, in a case where only the cell distribution area CDA having a low cell density exists on the bottom surface 31, it is assumed that it is difficult to distinguish between a location where the cell density is sparse and the void portion EA on the image.

As described above, the storage unit 75 stores information regarding the tip shape of the chip 10, for example, information such as the outer diameter and the wall thickness of the syringe tip portion 113 and the opening diameter of the tip opening t. The void portion EA formed by pressing the tip of the chip 10 is a trace substantially along the tip shape of the chip 10. Therefore, by referring to the information, it is easy to specify the void portion EA on the image. For example, the void portion EA can be detected by performing matching processing on an image including the void portion EA using a template corresponding to the tip portion 10T.

It is also assumed that a clear trace cannot be formed on the bottom surface 31 of the well 3 by simply pressing the tip of the chip 10 in the Z direction even if the information regarding the tip shape is referred to. In addition, the void portion EA close to a circle is not necessarily formed, and a case where it is difficult to detect the actual coordinate position PB based on the center of a circle as illustrated in FIG. 7B is also assumed. In this case, the trace creating unit 792 may be caused to execute the trace imparting process of pressing the tip of the chip 10 against the cell distribution area CDA and then horizontally moving the chip 10 in a predetermined direction. According to this trace imparting process, it is possible to form a longitudinal void portion in a predetermined direction in the cell distribution area CDA.

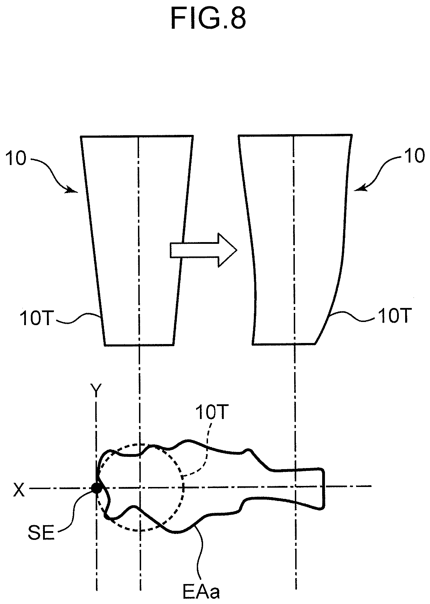

FIG. 8 is a diagram illustrating an example in which a longitudinal void portion EAa is formed in the cell distribution area by the horizontal movement of the chip 10. The trace creating unit 792 lowers the head 61 at a pitch of 5 µm, for example, and causes the tip portion 10T to approach the cell distribution area CDA. When it is confirmed from the image acquired by the camera unit 5 that the tip portion 10T (syringe tip portion 113) is in contact with and faces the cell distribution area CDA, the trace creating unit 792 moves the head 61 by, for example, about 50 µm in the X direction. With this operation, the contact mark of the tip portion 10T extends in the X direction, and the longitudinal void portion EAa in the X direction is formed. Such a void portion EAa has an advantage that it is easier to specify the void portion EAa on the image than a void formed by simply pressing the tip of the chip.

After the formation of the void portion EAa, the trace creating unit 792 causes an image including the void portion EAa to be acquired, and causes the image processing unit 74 to execute a process of obtaining the contour of the void portion EAa. In a case where the area of the void portion EAa is too small with respect to the area of the tip portion 10T, for example, in a case where the area is 50% or less, the trace creating unit 792 repeatedly executes the operation of lowering the head 61 by one pitch and further moving the head 61 in the X direction. The same operation is repeated until the area of the void portion EAa reaches a predetermined size.

In this case, the correction value calculating unit 793 handles the position corresponding to the starting end SE in the moving direction of the chip 10 in the longitudinal void portion EAa as the actual coordinate position PB. When the chip 10 is moved in a predetermined direction in a state where the tip portion 10T is in contact with the cell distribution area CDA or the bottom surface 31, the chip 10 is deformed as illustrated in FIG. 8. When the bending occurs, a positional relationship between the axial center of the head 61 and the tip portion 10T of the chip 10 is deviated. Therefore, when the terminal end in the moving direction of the chip 10 is set as the actual coordinate position PB, an error occurs. Since the bending hardly occurs in the chip 10 at the starting end SE, it is possible to suppress the intervention of the error in the actual coordinate position PB.

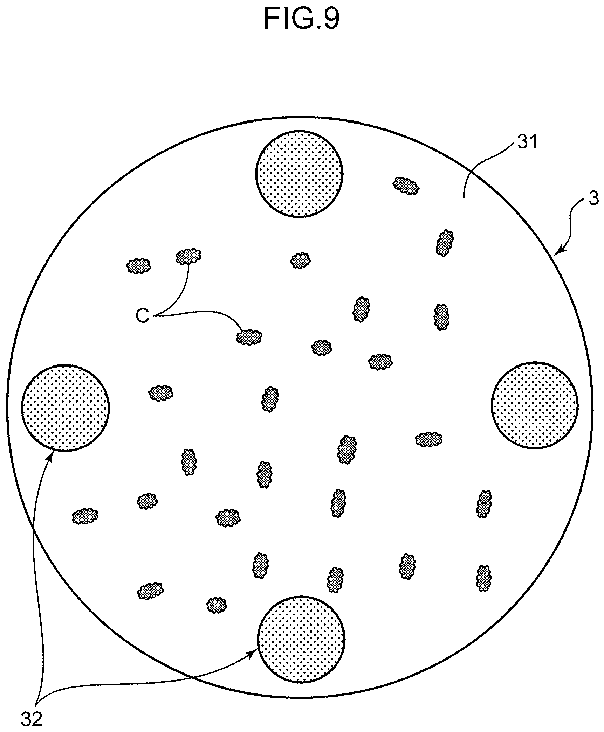

In the above embodiment, the example in which the cell distribution area CDA in which the cells C are actually distributed is set as the calibration area and the trace is imparted is shown, but a region other than the cell distribution area CDA may be set as the calibration area. FIG. 9 is a diagram illustrating another example of setting of the calibration area. On the bottom surface 31 of the well 3, a circular coating layer 32 (variable area) is provided at four locations near the outer peripheral edge. The coating layer 32 is a layer in which a trace can be formed by contact of the tip portion 10T of the chip 10. Examples of the coating layer 32 include an easily peelable ink coating layer and a coating film layer of a gel-like member.

In this case, the area setting unit 791 of the information processing unit 79 designates the coating layer 32 as the calibration area. The trace creating unit 792 presses the tip portion 10T of the chip 10 against the coating layer 32 to form a trace on the coating layer 32. The correction value calculating unit 793 specifies an actual coordinate position from the imparted trace, and derives a correction value from a difference between a target coordinate position set for imparting the trace and the actual coordinate position. Note that the member forming the calibration area is not limited to the coating layer 32, and may be any variable object as long as the state can be changed by contact with the tip portion 10T of the chip 10. For example, the calibration area may be constituted by minute objects such as beads or powder.

### [Control Flow of Cell Picking]

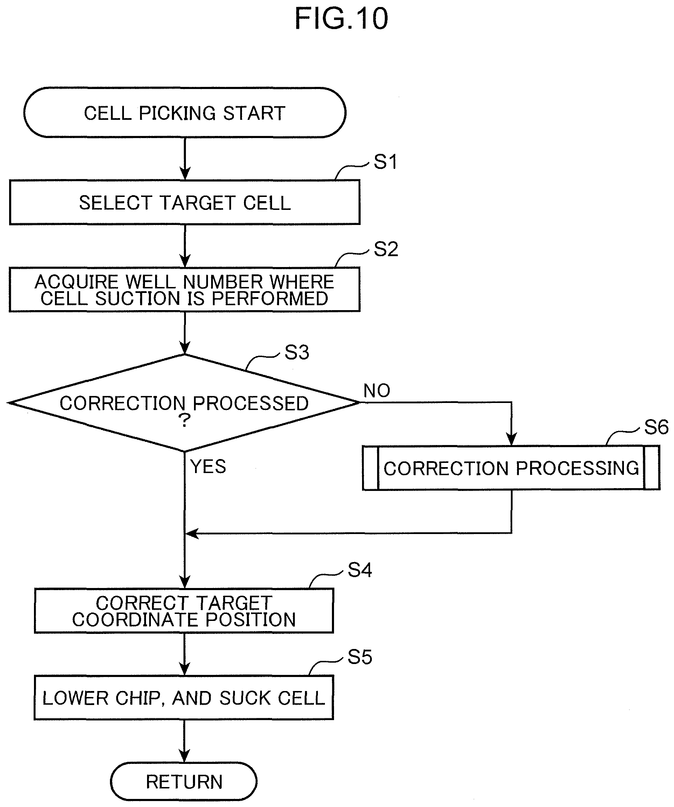

FIG. 10 is a flowchart illustrating the cell picking processing. The main control unit 78 receives selection of the target cell Ct to be moved from the culture plate 2 to the transfer destination plate 4 from the user via the input unit 76 (step S1). At the time of this selection, an image of the bottom surface 31 of the well 3, which is the cell culture surface, captured by the camera unit 5 is displayed on the display unit 77. The user specifies the target cell Ct while visually recognizing the display unit 77, and inputs the selection to the input unit 76.

The information processing unit 79 acquires the address of the well 3 in which the selected target cell Ct exists (step S2). For example, when the above-described 6-well plate is used as the culture plate 2, a well address assigned in advance to the well is acquired in order to specify in which well among the six wells the target cell Ct exists.

Subsequently, the information processing unit 79 confirms whether the correction processing has been executed for the chip 10 currently attached to the head 61 for the well 3 of the acquired address (step S3). The correction processing is a process of correcting the XYZ movement amount of the head 61 described above in consideration of the positional shift of the tip portion 10T of the chip 10, the height variation of the bottom surface 31 of the well 3, and the like. When the correction value has already been acquired for the well 3 accommodating the target cell Ct, that is, when the correction value is stored in the storage unit 75 (YES in step S3), the information processing unit 79 corrects the coordinate position of the target cell Ct specified on the image with the correction value (step S4).

The coordinate position corrected in step S4 becomes the coordinate position to be the movement target of the head 61. The shaft control unit 71 moves the head unit 6 in the XY directions so that the head 61 is directed to the coordinate position. After the XY movement, the head control unit 72 lowers the head 61 (Z movement) and generates a negative pressure in the tip opening t of the chip 10 to suck the target cells Ct (step S5). Thereafter, the head unit 6 is moved to the transfer destination plate 4. On the other hand, in a case where the correction value has not been acquired yet (NO in step S3), processing for acquiring the correction value is executed by the information processing unit 79 (step S6).

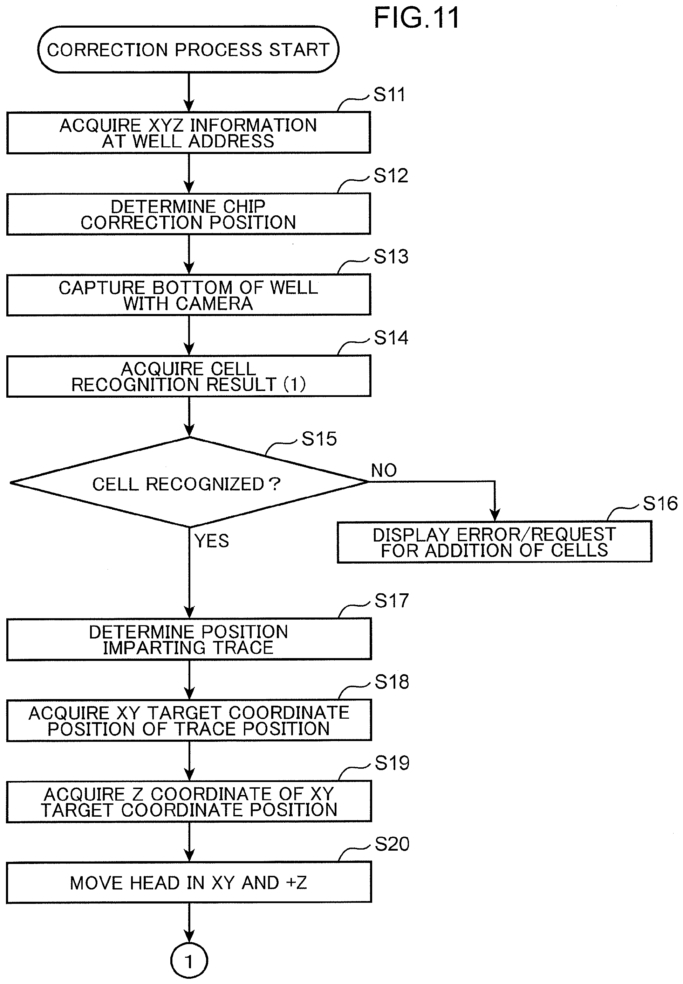

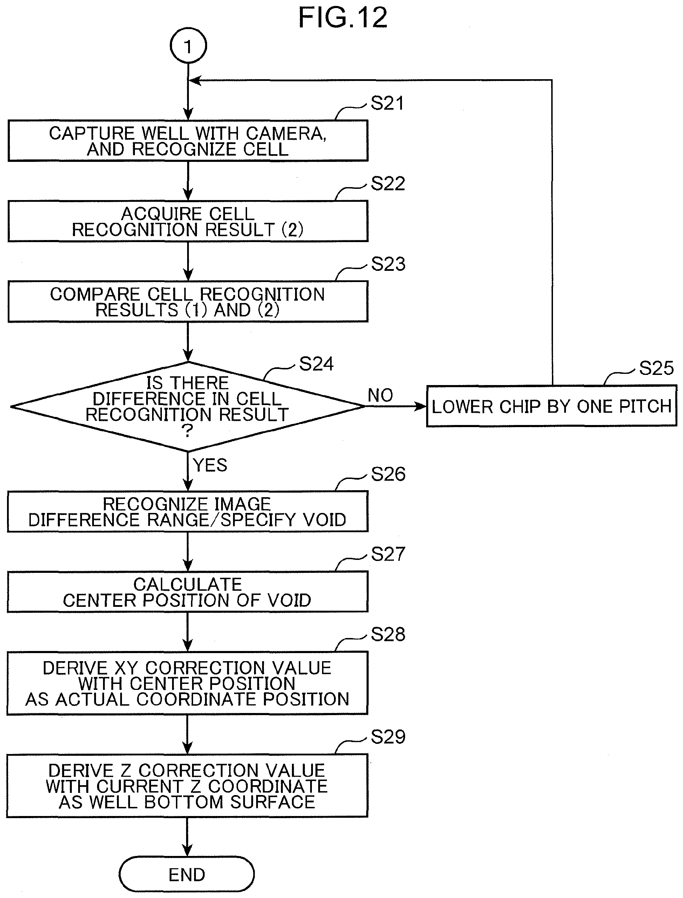

FIGS. 11 and 12 are flowcharts illustrating correction processing. The information processing unit 79 acquires XYZ information, which is information of the XY position and the height position of the well 3 at the address acquired in step S2, from the storage unit 75 (step S11). Next, the area setting unit 791 sets a chip correction position, which is a calibration area for imparting a trace to the chip 10, on the bottom surface 31 of the well 3 (step S12). The process of step S12 corresponds to the process of setting the chip correction positions TA1 to TA5 illustrated in FIG. 5.

Subsequently, the main control unit 78 causes the camera shaft drive unit 53 to move the camera unit 5 in the XY directions and causes the imaging control unit 73 to perform an imaging operation, whereby the image of the bottom surface 31 of the well 3 is acquired (step S13). The image processing unit 74 performs predetermined image processing on the acquired image, and performs recognition processing of the cells C distributed on the bottom surface 31 (step S14). Then, based on the cell recognition result, it is confirmed whether a cell group to an extent that a trace can be imparted by the chip 10, for example, the cell distribution area CDA illustrated in FIG. 5 is recognized (step S15). When the cell distribution area CDA is not confirmed (NO in step S15), the main control unit 78 displays an error on the display unit 77 and displays a message prompting addition of the cell C to the well 3 (step S16).

On the other hand, when the cell group is recognized on the bottom surface 31 (YES in step S15), the trace creating unit 792 determines a trace position where a trace is imparted by the chip 10 (step S17). The trace position is set in a region where cells other than the target cell Ct gather at the chip correction position determined in step S12. In the example of FIG. 5, for example, the trace position is set in an unused cell group around the target cell Ct1 in the cell distribution area CDA existing at the chip correction position TA3.

Next, the trace creating unit 792 acquires XY coordinate values of the trace position (step S18). This XY coordinate value corresponds to the target coordinate position PA illustrated in FIG. 7 and is an XY coordinate to be a movement target of the head 61 in the operation of imparting a trace. The trace creating unit 792 further acquires the Z coordinate of the XY coordinate position (step S19). When acquiring the Z coordinate, the trace creating unit 792 refers to the data of the height dimensional tolerance of the bottom surface 31 and the mechanical error data of the head unit 6 stored in the storage unit 75. The head 61 is moved in the XY directions based on the XYZ coordinate data obtained in steps S18 and S19, and is lowered (moved in Z) after the movement (step S20).

Subsequently, an image of the bottom surface 31 of the well 3 is acquired by the camera unit 5, and recognition processing of the cells C distributed on the bottom surface 31 is executed based on the image (step S21). The trace creating unit 792 acquires the cell recognition result of step S21 (step S22), and executes comparison processing with the cell recognition result previously acquired in step S14 (step S23). As this comparison processing, for example, image subtraction processing can be applied.

In a case where there is a difference between the two cell recognition results, the tip portion 10T of the chip 10 comes into contact with the cell distribution area CDA by lowering of the head 61 in step S20, and a trace is imparted. When there is no difference between the two cell recognition results (NO in step S24), no trace is imparted yet, and thus the head 61 is lowered so that the chip 10 is lowered by one pitch (for example, 5 µm) (step S25). Thereafter, the process returns to step S21 and is repeated. That is, while gradually lowering the height of the tip portion 10T, the degree of trace attachment is confirmed on the image.

On the other hand, when a difference between the two cell recognition results is confirmed (YES in step S24), the correction value calculating unit 793 specifies a location related to the difference, that is, a trace caused by the contact of the chip 10 on the image as the void portion EA illustrated in FIG. 7B (step S26). Furthermore, the correction value calculating unit 793 obtains the contour of the void portion EA and calculates the center position thereof, thereby obtaining the coordinates of the position where the tip portion 10T of the chip 10 actually comes into contact with the cell distribution area CDA (step S27). The coordinates become the actual coordinate position PB illustrated in FIG. 7B.

Subsequently, the correction value calculating unit 793 obtains a difference between the actual coordinate position PB acquired in step S28 and the target coordinate position PA specified in step S18 to derive an XY correction value for the movement of the head 61 (step S28). Furthermore, the correction value calculating unit 793 derives the Z correction value based on the Z height of the tip portion 10T when the contact trace of the chip 10 is confirmed (step S29). These XYZ correction values are stored in the storage unit 75 and used for correction in step S4 of FIG. 10.

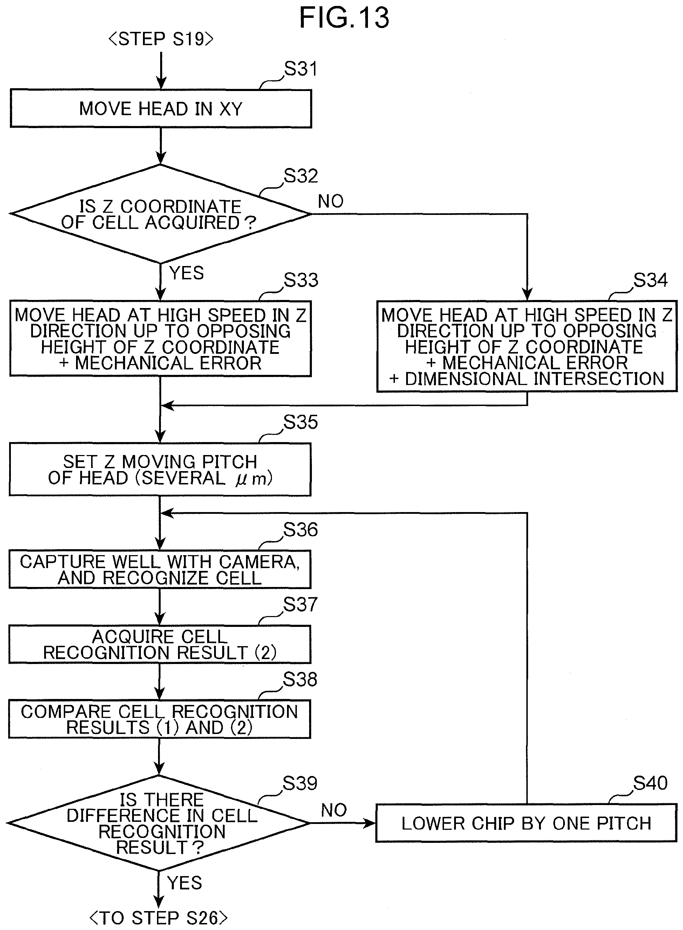

FIG. 13 is a flowchart illustrating correction processing according to a modification, and is an example of changing the lowering aspect of the head 61 depending on whether the Z coordinate of the cell C has been accurately acquired. The flowchart of FIG. 13 illustrates a process alternative to step 19 of FIG. 11 to step S26 of FIG. 12. In step S20 of the above embodiment, an example has been described in which the head 61 is simply sequentially moved in the XY directions and the Z direction. In the modification, after the head 61 is moved in the XY directions (step S31), the trace creating unit 792 determines whether the Z coordinate of the cell C has been accurately acquired by the imaging so far (step S32).

When the Z coordinate of the cell C has been accurately acquired (YES in step S32), the trace creating unit 792 sets the predetermined opposing height at which the lowering of the head 61 is switched from the high-speed lowering to the stepwise lowering to a height position obtained by adding the upper limit of the mechanical error of the head unit 6 to the Z coordinate of the cell C. Then, the trace creating unit 792 controls the head drive unit 64 through the head control unit 72 to move (lower at a high speed) the head 61 in the Z direction at a high speed until the tip 10T of the chip 10 reaches the opposing height (step S33).

On the other hand, when the Z coordinate of the cell C cannot be accurately acquired (NO in step S32), the trace creating unit 792 sets the opposing height to a height position obtained by adding the upper limit of the height dimensional tolerance of the bottom surface 31 and the upper limit of the mechanical error of the head unit 6 to the temporarily acquired Z coordinate of the cell C. Then, the trace creating unit 792 lowers the head 61 at a high speed until the tip 10T of the chip 10 reaches the opposing height (step S34).

Subsequent to step S33 or step S34, the trace creating unit 792 sets the Z moving pitch of the head 61 after the opposing height (step S35). For example, an instruction signal is imparted to the head control unit 72 so as to gradually lower the head 61 at a pitch of several µm.

Hereinafter, a process of causing the camera unit 5 to image the cell distribution area CDA and confirming the pressing trace of the chip 10 is performed every one pitch of lowering of the head 61. An image of the well 3 is acquired by the camera unit 5, and recognition processing of the cells C distributed on the bottom surface 31 is executed based on the image (step S36). The trace creating unit 792 acquires the cell recognition result of step S36 (step S37), and executes comparison processing with the cell recognition result previously acquired in step S14 (step S38). When there is no difference between the two cell recognition results (NO in step S39), the head 61 is lowered by one pitch (step S40). Thereafter, the process returns to step S36 and is repeated. On the other hand, when a difference between the two cell recognition results is confirmed (YES in step S39), the process proceeds to step S26 in FIG. 12. By performing the determination in step S32, the execution period of the time-consuming loop in steps S36 to S40 can be shortened.

### [Second Embodiment of Correction Control]

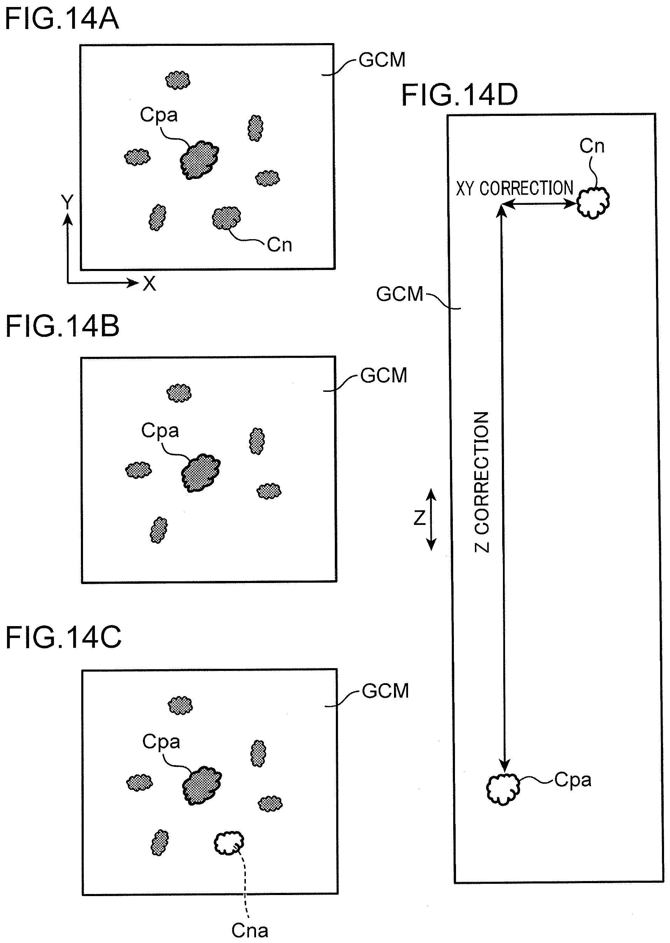

In the second embodiment, an example is shown in which the calibration area is a gel-like medium, and the actual coordinate position PB is obtained from the suction trace of the cell C sucked by the chip 10. FIGS. 14A to 14D are diagrams for explaining correction processing according to the second embodiment of the present invention. In the first embodiment, the picking of the cell C in which the liquid medium LCM is injected into the well 3 and cultured in contact with the bottom surface 31 is exemplified. The cells may be cultured in a gel-like medium GCM. The present invention can also be applied to transfer of cells in the gel-like medium GCM.

As illustrated in FIGS. 14A and 14D, a large number of cells including target cells Cpa to be sucked in the correction control and surrounding cells Cn are cultured in a gel-like medium GCM. The target cell Cpa and the surrounding cells Cn are separated in the XY directions and also exist at positions separated in the Z direction.

The area setting unit 791 of the information processing unit 79 sets a certain three-dimensional region in the gel-like medium GCM as the calibration area. Here, it is assumed that the target cell Cpa and the surrounding cells Cn are included in the calibration area. The trace creating unit 792 specifies XYZ coordinates of the target cell Cpa from the camera image, and determines the coordinates as a target coordinate position to be a movement target of the head 61. The target coordinate position is imparted to the shaft control unit 71 and the head control unit 72, the head 61 is moved to the XY coordinates of the target coordinate position, and the tip portion 10T of the chip 10 is lowered to the Z coordinate of the target coordinate position. Then, the suction operation of the chip 10 is performed.

FIG. 14B is an image of the gel-like medium GCM in the calibration area after the suction operation is performed. Although the suction operation is performed toward the target cell Cpa, the target cell Cpa remains in the gel-like medium GCM. On the other hand, it is found that the surrounding cells Cn have disappeared, and instead, the surrounding cells Cn have been sucked into the chip 10. When the cells are sucked in the gel-like medium GCM, a trace due to a cavity of a part where the cells exist temporarily appears. That is, as illustrated in FIG. 14C, the suction trace Cna remains after the surrounding cells Cn are sucked.

The correction value calculating unit 793 specifies the coordinate position of the suction trace Cna and treats this as an actual coordinate position. Then, the correction value calculating unit 793 obtains a difference between the target coordinate position, which is the coordinate of the target cell Cpa to be sucked, and the actual coordinate position, which is the coordinate of the suction trace Cna that has actually been sucked. Based on this difference, as illustrated in FIG. 13D, the XY correction value and the Z correction value for the movement of the head 61 are acquired.

### [Other Embodiments]

Although the embodiment of the present invention has been described above, the present invention is not limited to the above embodiment, and various modifications can be made. For example, in the first embodiment, an example has been described in which a target coordinate position to be a transfer destination of the head 61 for forming a trace is set in the cell distribution area CDA. Alternatively, one cell (or one bead) may be set as the target coordinate position, or the center of about two or three adjacent cells may be set as the target coordinate position. The correction value of the position coordinates of the head 61 may be derived based on the trace of the shift of the one cell as the chip 10 comes into contact with the one cell. Note that the correction value may be derived by treating the position of the cell with which the chip 10 has come into contact as a target coordinate position.

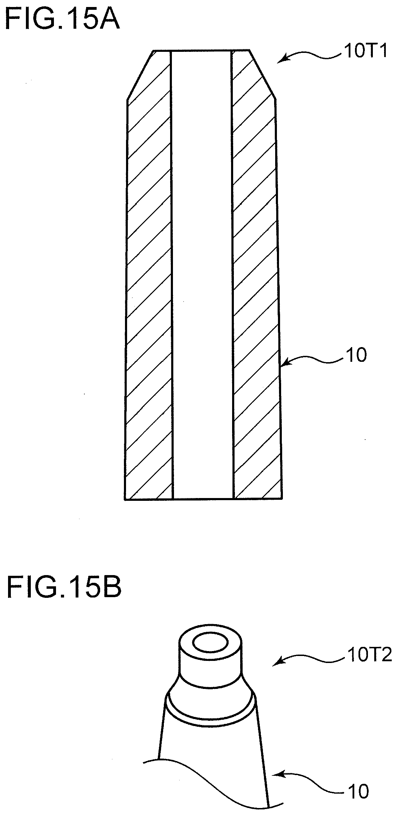

In addition, the tip portion 10T of the chip 10 desirably has a smaller outer diameter for accurate cell picking. FIGS. 15A and 15B illustrate a desirable shape of the tip portion of the chip 10. A tip portion 10T1 in FIG. 15A has a shape in which the outer peripheral surface is tapered in diameter toward the tip. A tip portion 10T2 in FIG. 15B has a shape reduced in diameter stepwise toward the tip. By using the chip 10 having the tip portions 10T1 and 10T2 reduced in diameter in this manner, it is possible to perform cell picking with higher accuracy. However, from the viewpoint of imparting a trace to the calibration area, since the tip of the chip 10 is reduced in diameter, the trace is also reduced, and it may be difficult to determine the trace on the image. Therefore, it is desirable to adopt the trace imparting operation accompanying the movement of the chip 10 in the X direction as illustrated in FIG. 8.

According to the cell transfer device S according to the present embodiment described above, the target coordinate position is imparted to the drive unit of the head 61 to operate the head 61, and the trace is actually imparted by the chip 10 to the calibration area of the well 3 of the culture plate 2. Then, the correction value of the coordinate position to be the movement target of the head 61 is acquired using the trace. That is, the deviation between the target coordinate position PA and the actual coordinate position PB is obtained based on the actual operation record of the head 61, and the correction value is derived. Therefore, even if there is a height variation of the bottom surface 31 of the well 3 or a positional shift of the tip portion 10T of the chip 10 attached to the head 61, an accurate correction value can be acquired. Therefore, a small target cell Ct having a size of several µm to several tens of µm can be accurately picked.

### [Inventions included in Above Embodiments]

The embodiments described above include the following inventions.

A cell transfer device according to one aspect of the present invention is a cell transfer device that transfers a specific target cell among cells accommodated in a first container to a second container, the cell transfer device including a camera configured to capture an image of a cell accommodated in the first container, a head to which a chip having a tip that sucks and discharges cells is attached, the head performing an operation of transferring the target cell to a position of the target cell in the first container based on an image captured by the camera, sucking the target cell into the chip, then moving to the second container, and discharging the target cell in the chip into the second container, and an information processing unit that corrects position coordinates at which the head exists, in which the information processing unit is configured to execute setting a calibration area in the first container and operating the head so that the chip imparts a predetermined trace to the calibration area with a predetermined location of the calibration area as a target coordinate position to be a transfer destination of the head, and specifying an actual coordinate position of the trace by causing the camera to image an area including the trace, and obtaining a difference between the target coordinate position and the actual coordinate position to acquire a correction value of the position coordinate of the head.

According to this cell transfer device, a target coordinate position is imparted to operate the head, and a trace is actually imparted to the calibration area of the first container with the chip. Then, the correction value of the coordinate position is acquired using the trace. That is, the deviation between the target coordinate position and the actual coordinate position is obtained based on the actual operation record of the head, and the correction value is derived. Therefore, even if there is a height variation of the culture surface of the first container or a positional shift of the tip of the chip attached to the head, an accurate correction value can be acquired. Therefore, a small target cell having a size of several µm to several tens of µm can be accurately picked.

In the cell transfer device, the information processing unit is desirably configured to execute setting the calibration area to a cell distribution area where cells other than the target cell gather in the first container, and pressing a tip of the chip against the cell distribution area as the trace to form a void in the cell distribution area.

According to this aspect, a trace is formed by pressing the tip of the chip against the cell distribution area. When the tip of the chip is pressed against an area in which cells are distributed to some extent, the cells existing in the pressed region are released to the periphery of the pressed region. As a result, the pressed region becomes a void area where there are no cells or the number of cells is extremely small. Such voids are easily confirmed by a camera image. That is, a trace that can be easily confirmed on the image can be formed by a simple operation of pressing the tip of the chip.

In the cell transfer device, the information processing unit can obtain a center position of the void based on an image including the void, and set the center position as the actual coordinate position.

The void formed by pressing the tip of the chip has a constant area. Therefore, it is desirable to specify any position of the void as the actual coordinate position. According to the above aspect, the actual coordinate position can be specified by a reasonable and simple method of obtaining the center position of the void.

In the cell transfer device, the information processing unit desirably refers to information regarding a tip shape of the chip when specifying the void from the image including the void.

Since there may be a cell distribution area having a low cell density in the first container, it may be difficult to distinguish the voids on the image. According to the above aspect, since the information regarding the tip shape of the chip, for example, the tip shape and the size is referred to, it is easy to specify the void formed by pressing the tip of the chip on the image.

In the cell transfer device, the information processing unit may press the tip of the chip against the cell distribution area and then move the chip in a predetermined direction to form a longitudinal void in the predetermined direction in the cell distribution area.

According to this aspect, the longitudinal void can be formed in the cell distribution area by the movement of the tip after the tip of the chip is pressed. Such a void has an advantage that it is easier to specify the void on the image than a void formed by simply pressing the tip of the chip.

In this case, the information processing unit desirably handles a position corresponding to a starting end in a moving direction of the chip in the longitudinal void as the actual coordinate position.

When the chip is moved in a predetermined direction, the chip is bent, and the positional relationship between the axial center of the head and the tip of the chip may be deviated. According to the above aspect, since the starting end in the moving direction of the chip is treated as the actual coordinate position, the influence of the above-described deviation can be eliminated.

In the cell transfer device, the tip of the chip is desirably pressed against the cell distribution area by lowering of the head, and the information processing unit desirably lowers the head at a first speed until the tip of the chip is positioned at a predetermined opposing height, and lowers the head at a second speed equal to or less than the first speed after the predetermined height.

In order to determine whether the chip imparts a trace to the cell distribution area, it is necessary to acquire an image before the tip of the chip is pressed against the cell in the cell distribution area and an image after the tip is pressed against the cell. However, the head may be lowered at a high speed up to a height at which the tip of the chip does not obviously abut on the cell. According to the above aspect, the head is lowered at a first speed until the opposing height, and then the head is lowered at a low second speed. Therefore, it is possible to shorten the tact of the process of giving a trace by pressing the chip.

In the cell transfer device, the information processing unit desirably lowers the head at a predetermined pitch after the predetermined height and causes the camera to execute an imaging operation at every one pitch of the lowering.

According to this aspect, the trace formed by the pressing of the tip of the head against the cell distribution area can be reliably captured.

In the cell transfer device, the first container desirably has a flat bottom surface, and cells are located on the bottom surface, and the information processing unit is desirably configured to execute setting when a height position of a cell has been accurately acquired, a height obtained by adding a mechanical error of the head to the height position of the cell to the opposing height, and setting a height obtained by adding a height dimensional tolerance of the bottom surface of the first container and a mechanical error of the head to a temporarily acquired height position of the cell to the opposing height when a height position of the cell has been temporarily acquired but accuracy is unknown.

According to this aspect, the lowering aspect of the head 61 is changed depending on whether the height position of the cell can be accurately acquired. Therefore, it is possible to reliably acquire images before and after the chip hits the cell while shortening the tact of the process of imparting a trace.

In the cell transfer device, the information processing unit may be configured to execute setting the calibration area to a variable area in which a variable object capable of changing a state by contact with the chip is arranged in the first container, and pressing the tip of the chip against the variable area as the trace to form a trace in the variable area.

According to this aspect, since a trace can be formed by pressing the tip of the chip against the variable area, it is possible to avoid pressing the tip of the chip against the cell in the first container.

In the cell transfer device, the information processing unit desirably acquires correction values in X and Y directions on an XY plane as correction values of the position coordinates of the head. Further, it is desirable to acquire a correction value in a Z direction orthogonal to the XY plane.

According to this aspect, the movement control of the head in the XY plane can be accurately performed. Furthermore, if the correction value in the Z direction is acquired, the three-dimensional movement control of XYZ of the head can be accurately performed.

According to the present invention, it is possible to provide a cell transfer device capable of accurately picking a target cell.

## Claims

1. A cell transfer device that transfers a specific target cell among cells accommodated in a first container to a second container, the cell transfer device comprising:
a camera configured to capture an image of a cell accommodated in the first container;
a head to which a chip having a tip that sucks and discharges cells is attached, the head performing an operation of transferring the target cell to a position of the target cell in the first container based on an image captured by the camera, sucking the target cell into the chip, then moving to the second container, and discharging the target cell in the chip into the second container; and
an information processing unit that corrects position coordinates at which the head exists,
wherein the information processing unit is configured to execute:
setting a calibration area in the first container and operating the head so that the chip imparts a predetermined trace to the calibration area with a predetermined location of the calibration area as a target coordinate position to be a transfer destination of the head; and
specifying an actual coordinate position of the trace by causing the camera to image an area including the trace, and obtaining a difference between the target coordinate position and the actual coordinate position to acquire a correction value of the position coordinate of the head.

2. The cell transfer device according to claim 1, wherein
the information processing unit is configured to execute:
setting the calibration area to a cell distribution area where cells other than the target cell gather in the first container; and
pressing a tip of the chip against the cell distribution area as the trace to form a void in the cell distribution area.

3. The cell transfer device according to claim 2, wherein the information processing unit obtains a center position of the void based on an image including the void, and sets the center position as the actual coordinate position.

4. The cell transfer device according to claim 2, wherein the information processing unit refers to information regarding a tip shape of the chip when specifying the void from the image including the void.

5. The cell transfer device according to claim 2, wherein the information processing unit presses the tip of the chip against the cell distribution area and then moves the chip in a predetermined direction to form a longitudinal void in the predetermined direction in the cell distribution area.

6. The cell transfer device according to claim 5, wherein the information processing unit handles a position corresponding to a starting end in a moving direction of the chip in the longitudinal void as the actual coordinate position.

7. The cell transfer device according to claim 2, wherein
the tip of the chip is pressed against the cell distribution area by lowering of the head, and
the information processing unit lowers the head at a first speed until the tip of the chip is positioned at a predetermined opposing height, and lowers the head at a second speed equal to or less than the first speed after the predetermined height.

8. The cell transfer device according to claim 7, wherein the information processing unit lowers the head at a predetermined pitch after the predetermined height and causes the camera to execute an imaging operation at every one pitch of the lowering.

9. The cell transfer device according to claim 7, wherein
the first container has a flat bottom surface, and cells are located on the bottom surface, and
the information processing unit is configured to execute:
setting when a height position of a cell has been accurately acquired, a height obtained by adding a mechanical error of the head to the height position of the cell to the opposing height; and
setting a height obtained by adding a height dimensional tolerance of the bottom surface of the first container and a mechanical error of the head to a temporarily acquired height position of the cell to the opposing height when a height position of the cell has been temporarily acquired but accuracy is unknown.

10. The cell transfer device according to claim 1, wherein
the information processing unit is configured to execute:
setting the calibration area to a variable area in which a variable object capable of changing a state by contact with the chip is arranged in the first container; and
pressing the tip of the chip against the variable area as the trace to form a trace in the variable area.

11. The cell transfer device according to any one of claims 1 to 10, wherein the information processing unit acquires correction values in X and Y directions on an XY plane as correction values of the position coordinates of the head.

12. The cell transfer device according to claim 11, wherein the information processing unit further acquires a correction value in a Z direction orthogonal to the XY plane as a correction value of the position coordinate of the head.
